# EUROPÄISCHE PATENTSCHRIFT

(11) **EP 0 476 217 B1**
(45) Veröffentlichungstag und Bekanntmachung des Hinweises auf die Patenterteilung: **22.06.1994**
(21) Anmeldenummer: 91101429.8
(22) Anmeldetag: 04.02.1991
(51) Int. Cl.: C05F 17/02, C12M 1/00, C05F 17/00

(54) **Anlage zur Verwertung organischer Stoffe**
Apparatus for adding value to organic material
Dispositif de valorisation de matière organique

(30) Priorität: 18.09.1990 CH 3015/90
(43) Veröffentlichungstag der Anmeldung: 25.03.1992
(73) Patentinhaber: Schmid, Walter, CH-8152 Opfikon (CH)
(72) Erfinder: Schmid, Walter, CH-8152 Opfikon (CH); Widmer, Christian, CH-4051 Basel (CH); Wellinger, Arthur, Dr., CH-8357 Guntershausen (CH)
(74) Vertreter: Feldmann, Clarence Paul

(56) Entgegenhaltungen:
- EP-A- 205 721
- EP-A- 0 131 319
- EP-A- 0 241 357
- WO-A-82/02059
- CH-A- 100 710
- DE-A- 2 535 756
- DE-A- 2 924 387
- DE-A- 3 117 638
- DE-A- 3 228 895
- GB-A- 2 208 645
- US-A- 4 203 755
- Seminarbericht "Energy from biomass and wastes, Lake Buena Vista, Florida, USA, 30.01.90

## Beschreibung

Die vorliegende Erfindung betrifft eine Anlage zur Verwertung von organischen Feststoffen unter Gewinnung von Kompost und Biogas, bestehend aus einem, mit einem Einfüllstutzen, einem Auslassstutzen, sowie einem mit einem um seine Längsachse drehbaren Rührwerk versehenen Fermenter zur anaeroben Vergärung der organischen Feststoffe und einer dem Fermenter vorgeordneten Zerkleinerungsvorrichtung.

Aus der DE-A-31 52 609 ist ein Verfahren zur Verwertung von Hausmüll und anderen organischen Stoffen bekannt, bei welchem der angelieferte Hausmüll in einer zugeordneten Sortieranlage von den anorganischen Stoffen, wie zum Beispiel von Glas, Kunststoffen, Metallen oder dergleichen getrennt wird. Die organischen Stoffe werden mittels einem Förderband einer Dosiervorrichtung und von dort einem Mischbehälter zugeführt, in welchem die Stoffe von einem Rührwerk unter Zufuhr einer Flüssigkeit entsprechend gemischt werden. Anschliessend werden die Stoffe über einen Grob- und einen Feinzerkleinerer einem Wartebehälter zugeführt. Das Gemisch wird anschliessend über einen Wärmeaustauscher in einen Reaktionsbehälter gefördert, in welchem der Abbau der organischen Stoffe im wesentlichen durch Bakterien erfolgt.

Aus der DE-A-25 35 756 ist eine Anlage gemäss Oberbegriff des Patentanspruches 1 bekannt, wobei es sich hier um eine Anlage für ein Nassverfahren handelt, welches mit einem geneigten Fermenter arbeitet. Das Rührwerk dient nicht nur der Vermengung, sondern insbesondere auch dazu, die Bildung einer Schwimmdecke zu vermeiden. Um Feststoffe in die Flüssigphase einzutragen, ist im feststehenden Fermenter ein Einfülltrichter mit Förderschnecke vorgesehen, die gleichzeitig mit einer Zerkleinerungsvorrichtung in der Art eines Mixers ausgerüstet ist.

In der Anlage gemäss DE-A-32 28 895 wird mit einem geneigten, rotierenden Fermenter gearbeitet. Auch dieser Fermenter wird mit einem fliessfähigen Medium als Gemisch aus fein zerkleinertem Müll und Klärschlamm beschickt. Ueber die Art der Eintragung erfolgt keine Aussage. Zur Impfung des Fermenters wird diskontinuierlich in einem nicht geschlossenen System Faulgut vom Ausgang des Fermenters wieder in den Fermenter direkt eingebracht.

Es ist die Aufgabe der vorliegenden Erfindung, eine Anlage der eingangsgenannten Art aufzuzeigen, die einen Betrieb ermöglicht, mit direkter Rückführung von Faulgut vom Auslassstutzen des Fermenters zur Speiseleitung des Fermenters, um so eine wirtschaftliche Wiederverwertung von organischen Stoffen zu erreichen.

Diese Aufgabe löst eine Anlage mit dem Merkmalen des Patentanspruches 1.

Weitere Merkmale der Erfindung ergeben sich aus der folgenden Beschreibung in Verbindung mit den in der Zeichnung dargestellten Ausführungsbeispielen und den Patentansprüchen.

Die Erfindung wird nachstehend unter Bezugnahme auf die Zeichnung näher beschrieben. Es zeigt:
- Figur 1: ein als Gesamtansicht dargestelltes erstes Ausführungsbeispiel einer als Fliess-Schema dargestellten Anlage zur Verarbeitung und Verwertung organischer Stoffe, und
- Figur 2: ein Teilstück eines zweiten Ausführungsbeispiels einer als Fliess-Schema dargestellten Anlage zur Verarbeitung und Verwertung organischer Stoffe.

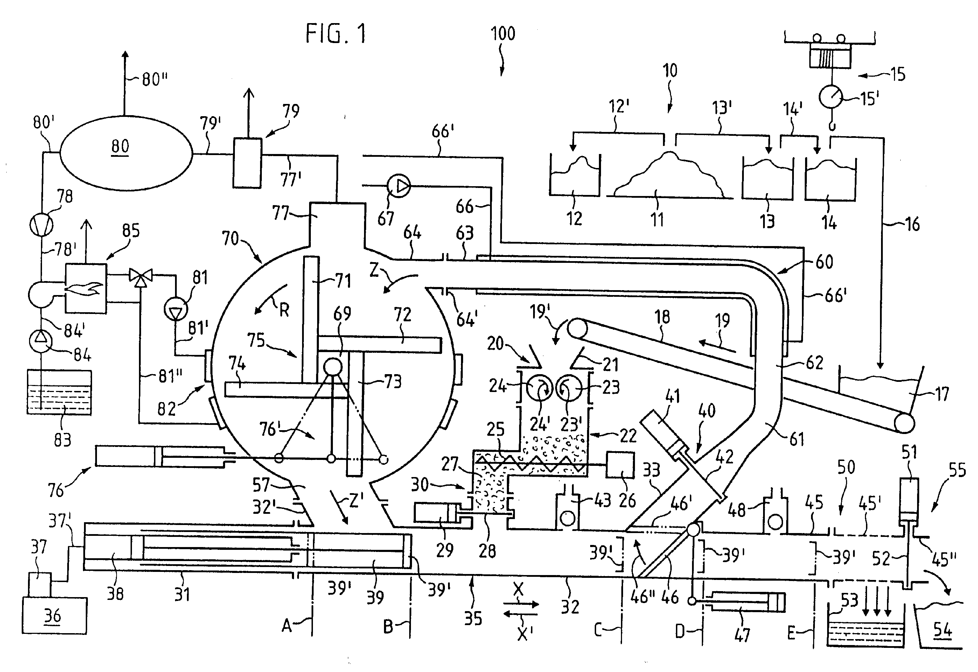

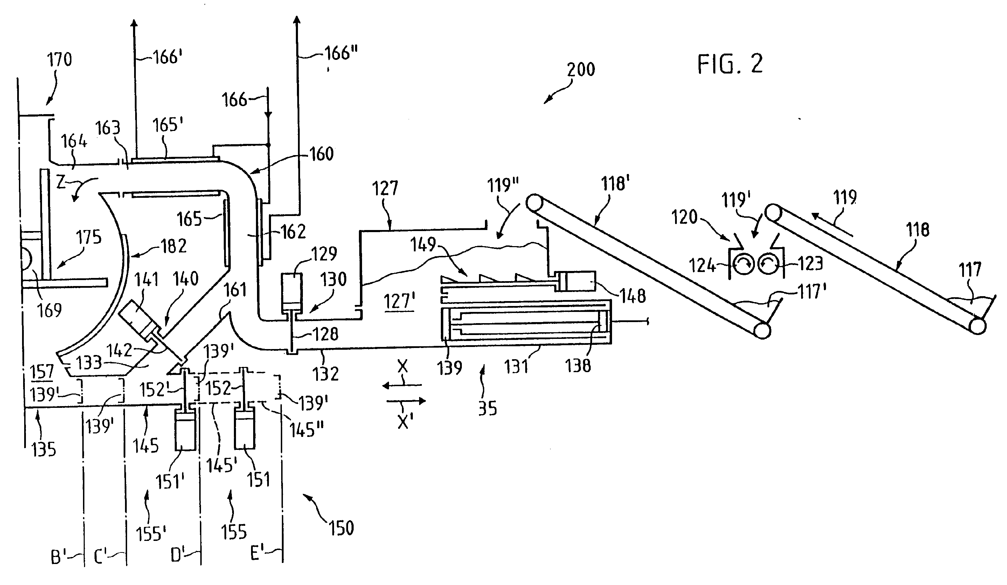

Figur 1 zeigt als erstes Ausführungsbeispiel eine im wesentlichen als Fliess-Schema dargestellte und in der Gesamtheit mit 100 bezeichnete Anlage und Figur 2 zeit ein Teilstück einer mit 200 bezeichneten Anlage. Die beiden zur Verarbeitung und Verwertung von organischen Feststoffen ausgebildeten Anlagen 100 und 200 werden nachstehend im Einzelnen beschrieben. Im Anschluss an die Figuren-Bezeichnung folgt die Beschreibung der Arbeitsweise der beiden Anlagen 100 und 200.

Die Anlage 100 umfasst im wesentlichen eine Sortieranlage 10, eine Zerkleinerungs-Vorrichtung 20, einen Förderschacht 35, eine Austrags-Vorrichtung 50, eine Speiseleitung 60, einen mit einem Rührwerk 75 versehenen Fermenter 70, einen dem Fermenter 70 entsprechend zugeordneten Heizkessel 85 sowie einen als Gas-Zwischenspeicher ausgebildeten Behälter 80. Die vorstehend aufgeführten Elemente werden nachstehend im einzelnen beschrieben.

Die Sortieranlage 10 hat eine beispielsweise behälterartig ausgebildete Sammelstelle 11, sowie entsprechend zugeordnete Behälter 12,13 und 14. In der Sammelstelle 11 werden die angelieferten Stoffe in organische und anorganische Stoffe sortiert.

Die anorganische Stoffe werden in Pfeilrichtung 12′ dem Behälter 12 und von dort einer nicht dargestellten Deponie zugeführt.

Die aussortierten, organischen Stoffe werden in Pfeilrichtung 13′ dem als Zwischenlager vorgesehenen Behälter 13 und von dort in Pfeilrichtung 14′ dem Behälter 14 zugeführt. Die organische Stoffe können aber auch von der Sammelstelle 11 direkt dem Behälter 14 zugeführt werden.

Mittels einem Fördergerät 15 werden die organischen Stoffe von dem Behälter 14 in Pfeilrichtung 16 einem weiteren, mit einem Förderband 18 in Wirkverbindung stehenden Behälter 17 zugeführt. Das Fördergerät 15 ist beispielsweise mit einer Waage 15′ versehen, mittels welcher die organischen Stoffe in bestimmte Mengen, beispielsweise in entsprechend zu verarbeitende Tagesportionen unterteilt werden können. Von einem dem Behälter 17 zugeordneten Transport-und Förderband 18 werden die organischen Stoffe in Pfeilrichtung 19,19′ einer Zerkleinerungs-Vorrichtung 20 zugeführt. Die Zerkleinerungs- Vorrichtung 20 umfasst einen mit einem Trichter 21 versehenen Behälter 22, in welchem zwei Rollenkörper 23 und 24 gelagert sind.

Die Rollenkörper 23,24 sind am äusseren Umfang mit nicht dargestellten Zerkleinerungselementen versehen, mittels welcher die in Pfeilrichtung 19′ zugeführten organischen Stoffe entsprechend zerkleinert werden. Die beiden Rollenkörper 23,24 sind in dem Behälter 22 gelagert und werden von AntriebsMotoren gegenläufig in Pfeilrichtung 23′, 24′ angetrieben.

Im unteren Bereich des Behälter 22 ist mindestens eine von einem Motor 26 angetriebene Förderschnecke 25 angeordnet und gelagert. Mittels der Förderschnecke 25 werden die zerkleinerten organischen Stoffe in eine schleusenartig ausgebildete Frischgutzuführung 27 des Behälters 22 transportiert und gepresst. Die Frischgutzuführung 27 wird im unteren Bereich von einem schieberartig ausgebildeten und von einer ersten Kolben/Zylindereinheit 29 betätigbaren Schliesselement 28 verschlossen. Die Frischgutzuführung 27 und das Schliesselement 28, sowie die Kolben/Zylindereinheit 29 bilden zusammen eine in der Gesamtheit mit 30 bezeichnete erste Schleuse.

Ueber die erste Schleuse 30 steht die Frischgutzuführung mit dem kanalartig ausgebildeten und in der Gesamtheit mit 35 bezeichneten Förderschacht in Verbindung. Der Förderschacht 35 umfasst ein erstes Teilstück 31, ein damit verbundenes zweites Teilstück 32, sowie ein drittes Teilstück 33.

In dem ersten Teilstück 31 des Förderschachtes 35 ist ein mit einer zweiten Kolben/Zylindereinheit 38 in Wirkverbindung stehendes Förderelement 39 angeordnet, welches mittels der Kolben/Zylindereinheit 38 in Pfeilrichtung X,X′ verschiebbar in dem Förderschacht 35 angeordnet ist. Das schematisch dargestellte Förderelement 39 ist beispielsweise als Kolbenpumpe ausgebildet, welche mit einem Hydraulik-Aggregat 36, sowie mit einem Antrieb 37 über eine Leitung 37′ wirkverbunden ist.

Das Förderelement 39 ist im Förderschacht 35 für verschiedene Funktionsschritte in verschiedene Stellungen positionierbar. Die einzelnen Stellungen A,B,C,D und E des Förderelementes 39 in den Schacht-Teilstücken 31,32, sowie in einem Schacht- Teilstück 45 einer Austragsvorrichtung 50 sind jeweils durch die gestrichelt dargestellte Förderelement-Vorderkante 39′ bezeichnet.

Im zweiten Teilstück 32 des Förderschachtes 35 mündet einerseits der Auslassstutzen 57 des Fermenters 70 über den Anschluss 32′ und andererseits die Frischgutzuführung 27. Das zweite Teilstück 32 des Förderschachtes 35 endet bei einer Klappe 46, die als Weiche dient. Somit wird hierin Frischgut via der Weiche 46 in die Speiseleitung 60 gefördert oder Faulgut aus dem Fermenter 70 bei entsprechender Stellung 46′ der Klappe 46 in die Austragsvorrichtung 50 geschoben.

Der unterste Bereich 33 der Speiseleitung 60 zweigt spitzwinklig vom Förderschacht 35 ab. Zur Steuerung der Anlage ist auch im untersten Bereich 33 der Speiseleitung 60 eine Schleuse 40, bestehend aus einer Kolben/Zylindereinheit 41 und einem Schieber 42, angeordnet. Schliesslich ist am zweiten Förderschachtabschnitt 32 auch noch ein Entlüftungsventil 43 symbolisch dargestellt. Die die Weiche bildende Klappe 46 ist wiederum mittels einer Kolben/Zylindereinheit 47 betätigbar.

Die Austragsvorrichtung 50 umfasst den mit einem Be- und Entlüftungsventil 48 versehenen Austragsschacht 45, dessen Teilstücke 45′ und 45˝ die Verlängerung des Förderschachtes bilden. Zwischen den beiden Austragsschacht-Teilstücken 45′, 45˝ ist eine dritte Schleuse 55 angeordnet, welche mit einem von einer Kolben/Zylindereinheit 51 betätigbaren Schliesselement 52 versehen ist. Dem einen, als Entwässerungselement ausgebildeten Schacht-Teilstück 45′ ist ein Aufnahme-Behälter 53 und dem zweiten Schacht-Teilstück 45˝ ist ein Aufnahmebehälter 54 für das entwässerte Faulgut zugeordnet.

Die beheizte Speiseleitung 60 umfasst ein erstes, ein zweites, sowie ein drittes Leitungsstück 61,62 und 63. Die Speiseleitung 60 ist mit dem Leitungsstück 63 an einem mit einem Flansch 64′ versehenen Einfüll-Stutzen 64 des Fermenter 70 angeordnet und befestigt. An dem zweiten und dritten Leitungsstück 62,63 ist weiterhin ein beheizbarer Mantel 65 angeordnet, welcher unter Zwischenschaltung einer Pumpe 67 über Leitungen 66, 66' mit dem Heizkessel 85 in Verbindung steht.

In dem horizontal liegend angeordneten Fermenter 70 ist das in der Gesamtheit mit 75 bezeichnete Rührwerk angeordnet.

Das Rührwerk 75 umfasst einen den Fermenter in axialer Richtung durchdringenden und um seine Längsachse drehbar gelagerten Achskörper 69. Der Achskörper 69 ist mit mehreren, in axialer, sowie in radialer Richtung versetzt zueinander angeordneten Rührelementen 71,72,73 und 74 versehen.

Der Achskörper 69 ist an einem Antrieb 76 wirkverbunden. Mittels dem beispielsweise als Kolbenantrieb ausgebildeten und über ein Hebelgestänge 76' mit dem Achskörper 69 wirkverbundenen Antrieb 76 wird das Rührwerk 75 schrittweise in Pfeilrichtung R angetrieben.

Um den Fermenter 70 ist ein beheizbarer Mantel 82 angeordnet, welcher über Leitungen 81′,81˝ und einer zwischengeschalteten Pumpe 81 mit dem Heizkessel 85 in Verbindung steht. Dem Heizkessel 85 ist ein Tank 83 zugeordnet, aus welchem über eine Leitung 84' unter Zwischenschaltung einer Pumpe 84 entsprechend Brennstoff zugeführt wird.

An seiner Oberseite ist der Fermenter 70 mit mindestens einem Stutzen 77 versehen, an welchem über ein Ventil 79 die Leitung 77′ angeschlossen ist. Das Ventil 79 ist über eine Leitung 79′ mit dem Gas-Zwischenbehälter 80 und dieser für die Zufuhr von gasförmigem Brennstoff über eine Leitung 80′ unter Zwischenschaltung eines Gebläses 78 über eine Leitung 78′ mit dem Heizkessel 85 verbunden.

Das Gas im Behälter 80 kann auch über eine Leitung 80˝ einer nicht dargestellten Energieversorgungs-Anlage zugeführt werden.

Figur 2 zeigt als zweites Ausführungsbeispiel ein Teilstück der schematisch dargestellten und in der Gesamtheit mit 200 bezeichneten Anlage. Die in Figur 2 nicht dargestellten Elemente sind im wesentlichen analog der in Verbindung mit Figur 1 beschriebenen Anlage 100 ausgebildet und angeordnet.

Die Anlage 200 umfasst ein erstes sowie ein zweites Transport- und Förderband 118,118′, eine dazwischen angeordnete Zerkleinerungs-Vorrichtung 120, einen Förderschacht 135, eine Speiseleitung 160, einen Fermenter 170 mit Heizmantel 182, sowie eine dem Fermenter 170 zugeordnete Austrags-Vorrichtung 150. Im Fermenter 170 ist ein Rührwerk 175 mit Achskörper 169 angeordnet.

Dem ersten Förderband 118 ist ein Behälter 117 zugeordnet, von welchem die organische Stoffe in Pfeilrichtung 119 und 119′ der mit zwei gegenläufig angetriebenen Rollenkörpern 123, 124 versehenen Zerkleinerungs-Vorrichtung 120 zugeführt werden. Die zerkleinerten Stoffe werden einem Behälter 117′ und von dort über das zweite Förderband 118′ einem Sammelbehälter 127 gemäss Pfeilrichtung 119˝ zugeführt.

Der Sammelbehälter 127 ist an einem Teilstück 132 der Frischguteintragung 235 angeflanscht. Dem Sammelbehälter 127 ist eine von einer Kolben/Zylindereinheit 148 betätigbare Eintragungs-Vorrichtung 149 zugeordnet, mittels welcher die Stoffe in die Frischgutzuführung 127′ geschoben und gepresst werden. In einem Teilstück 131 der Frischguteintragung 235 ist ein in Pfeilrichtung X,X′ linear verschiebbares Schubelement 139 angeordnet, welches mit einer Kolben/Zylindereinheit 138 in Wirkverbindung steht.

Die Frischguteintragung 235 ist an der Speiseleitung 160 angeflanscht. Zwischen den beiden Teilstücken 132 und 162 ist eine Schleuse 130 angeordnet, welche ein von einer Kolben/Zylindereinheit 129 betätigbares Schliesselement 128 aufweist.

Die beispielsweise mit zwei beheizten Mantelteilen 165, 165′ versehene Speiseleitung 160 ist im oberen Bereich mit einem Teilstück 163 an einen Einfüllstutzen 164 des Fermenters 170 und im unteren Bereich mittels einer Rückkoppelung 161 und einem Zweigstück 133 an einen Austragsschacht 145 angeflanscht. Zwischen diesen beiden Teilen 161 und 133 ist eine Schleuse 140 angeordnet, welche ein von einer Kolben/Zylindereinheit 141 betätigbares Schliesselement 142 aufweist.

Die Speiseleitung 160 wird über Zuführ-und Rückflussleitungen 166,166′ und 166˝ mit Mantelteilen 165, 165′ entsprechend beheizt.

Im unteren Bereich ist der Fermenter 170 mit mindestens einem Auslass-Stutzen 157 versehen, welcher mit einem Förderschacht 135 in Verbindung steht. Der an dem Förderschacht 135 anschliessende Austragsschacht 145 umfasst ein erstes Teilstück 145′ sowie ein zweites Teilstück 145˝, wobei zwischen dem Austragsschacht 145 und dem ersten Teilstück 145′ sowie dem ersten Teilstück 145′ und dem zweiten Teilstück 145˝ jeweils eine Schleuse 155 beziehungsweise 155′ angeordnet ist. Die Schleuse 155 hat ein von einer Kolben/Zylindereinheit 151 betätigbares Schliesselement 152 und die beispielsweise analog ausgebildete Schleuse 155′ hat ein von einer Kolben/Zylindereinheit 151′ betätigbares Schliesselement 152′.

Im Förderschacht 135 sowie im Austragsschacht 145 der Austrags-Vorrichtung 150 ist ein durch die gestrichelten Linien dargestelltes Förderelement angeordnet, welches für verschiedene Funktionsschritte linear verschiebbar und in verschiedenen Stellungen positionierbar ist.

Die wesentlichen Arbeitsschritte der Anlage 100 werden nachstehend beschrieben:

Der zerkleinerte von der Förderschnekce 25 in die Frischgutzuführung 27 gepresste organische Stoffe wird nachstehend als sogenanntes Frischgut und der nach Durchlauf durch den Fermenter 70 als sogenanntes Faulgut bezeichnet. In einer ersten Phase gelangt das von der Förderschnekce 25 zusammengepresste Frischgut bei geöffneter Schleuse 30 in das Teilstück 32 des Förderschachtes 35 und wird anschliessend vom dem Förderelement 39, beispielsweise durch eine mit der Schleuse 30 in Wirkverbindung stehenden Steuerung, in Pfeilrichtung X transportiert. Dieser Vorgang wird bei geöffneter Schleuse 40 und bei geschlossener Austragsvorrichtung 50 zum Beispiel schrittweise mehrfach wiederholt.

Die weitere Zufuhr des Frischgutes kann entsprechend gesteuert werden. Anschliessend wird das in der Speiseleitung 60 befindliche Frischgut erwärmt. Bei geschlossener Schleuse 30 und geöffneter Schleuse 40. fördert das Förderelement 39 neues Frischgut, so dass gleichzeitig das in der Speiseleitung 60 befindliche, erwärmte Frischgut in Pfeilrichtung Z in den ebenfalls beheizten Fermenter 70 gelangt.

Das in dem Fermenter 70 entstehende Gas wird über die am Stutzen 77 angeordnete und mit dem Innenraum des Fermenters 70 in Verbindung stehende Leitung 77′ dem Gas-Zwischenspeicher 80 zugeführt, so dass ein Aufblähen des im Fermenter 70 befindlichen Faulgutes verhindert wird. Von dem Gas-Zwischenspeicher 80 wird das Gas zur Energie-Erzeugung dem Heizkessel 85 zugeführt. Das im Fermenter 70 bei dem Gärungsprozess entstehende Gas kann somit in vorteilhafter Weise als Prozessenergie für den Betrieb des Heizkessels 85 verwendet werden.

Das Gas im Behälter 80 kann jedoch auch über ein Leitung 80˝ einer anderen, nicht dargestellten Energieversorgungs-Anlage zugeführt werden.

In einer Zwischenphase wird das Förderelement 39 in die den Auslass-Stutzen 57 des Fermenters 70 öffnende Stellung A zurückgefahren, so dass bei geschlossener Schleuse 30 aus dem Fermenter 70 eine bestimmte Menge des Faulgutes als sogenanntes Bakterien-Impfgut in das Teilstück 32 des Förderschachtes 35 transportiert wird.

Das Bakterien-Impfgut wird von dem Förderelement 39 in Pfeilrichtung X bis in die Stellung C gedrückt und dem im Teilstück 32 und dem untersten Bereich 33 der Speiseleitung befindlichen Frischgut zugeführt und anschliessend bei geöffneter Schleuse 40 in die Speiseleitung 60 gedrückt. Bei diesem Vorgang wird gleichzeitig die in der Speiseleitung befindliche Menge in Pfeilrichtung Z dem Behälter 70 zugeführt, während das mit dem Bakterien-Impfgut zusammengepresste Frischgut in der Speiseleitung 60 erwärmt wird.

In der Endphase, in welcher das Förderelement 39 in Pfeilrichtung X′ in die Stellung A zurückgefahren ist, wird das Faulgut aus dem Behälter in das Teilstück 32 gefördert und anschliessend von dem Förderelement 39, bei geöffneter Klappe 46 (gestrichelte Stellung), in die Austragsvorrichtung 50 geschoben. Bei diesem Vorgang bleibt zuerst die Schleuse 55 verschlossen, so dass in dieser Phase bei vorbestimmtem und entsprechend überwachtem Druck des Förderelementes 39 das Faulgut zusammengepresst und entwässert wird. Das Wasser wird von dem zugeordneten Behälter 53 aufgenommen. Sobald das Schubelement 39 die Stellung E erreicht hat, wird die Schleuse 55 geöffnet, so dass das zusammengepresste Faulgut in den zugeordneten Behälter 54 fällt. Vor dem Zurückfahren des Schubelementes 39 wird die Schleuse 55 der Austragsvorrichtung 50 wieder geschlossen.

Das nach dem vorstehend beschriebenen Verfahren gewonnene Faulgut kann anschliessend bei der Weiterverwertung als Zuschlags-Stoff für Kompost, Blumenerde oder dergleichen verwendet werden.

Abweichend von der vorstehend anhand der Anlage 100 gemäss Figur 1 beschriebene Arbeitsschritten wird bei der Anlage 200 gemäss Figur 2 der zerkleinerte organische Stoff im wesentlichen direkt von dem Förderelement 139 in die beheizte Speiseleitung 160 gedrückt.

Hierbei gelangt das von der Eintragungsvorrichtung 149 zusammengepresste Frischgut bei geöffneter Schleuse 130 in das Teilstück 132 der Frischguteintragung 235 und wird anschliessend von dem Förderelement 139, beispielsweise durch eine mit der Schleuse 130 in Wirkverbindung stehende Steuerung, in Pfeilrichtung X transportiert. Dieser Vorgang wird bei geschlossener Schleuse 140, zum Beispiel schrittweise, mehrfach wiederholt.
Die weiteren Arbeits-und Verfahrensschritte der Anlage 200 sind im wesentlichen mit den vorstehend in Verbindung mit der Anlage 100 gemäss Figur 1 beschriebenen Arbeits- und Verfahrensschritten identisch.

## Patentansprüche

1. Anlage zur Verwertung von organischen Feststoffen unter Gewinnung von Kompost und Biogas, bestehend aus einem, mit einem Einfüllstutzen (64,164), einem Auslassstutzen (57, 157) sowie einen mit einem um seine Längsachse drehbaren Rührwerk (75,175) versehenen Fermenter (70,170) zur Vergärung organischer Feststoffe und einer dem Fermenter zugeordneten Zerkleinerungsvorrichtung (20), dadurch gekennzeichnet, dass über einer geheizten Speiseleitung (60,160) der horizontale Fermenter (70,170) mit Frischgut füllbar ist und über einen am Auslassstutzen (57,157) des Fermenters anschliessenden Förderschacht direkt oder indirekt mittels Schleuse (30,40, 130,140) Faulgut dem Frischgut in der Speiseleitung beigebbar ist, so dass ein insgesamt in sich geschlossener Prozesskreislauf gebildet ist, wobei der Förderschacht (35, 135) in einen Austragsschacht (45,145) einer Austragsvorrichtung übergeht.

2. Anlage nach Anspruch 1, dadurch gekennzeichnet, dass der unterste Bereich (33,133) der Speiseleitung (60,160) eine Abzweigung des Förderschachtes (35,135) bildet.

3. Anlage nach Anspruch 2, dadurch gekennzeichnet, dass eine Frischgutzuführung (27) das Frischgut vor der Abzweigung zur Speiseleitung (60) via den Förderschacht (35) indirekt in die Speiseleitung (60) einleitet.

4. Anlage nach Anspruch 2, dadurch gekennzeichnet, dass eine Frischgutzuführung (127) das Frischgut nach der Abzweigung des untersten Bereiches (133) der Speiseleitung vom Förderschacht direkt in die Speiseleitung (60) einleitet.

5. Anlage nach Anspruch 1, dadurch gekennzeichnet, dass im Förderschacht (35,135) ein hydaulisch betätigbares Förderelement (39) angeordnet ist, mittels welchem ein im Fermenter (70,170) erzeugtes Faulgut als Bakterien-Impfgut im Förderschacht (35,135) transportierbar ist.

6. Anlage nach den Ansprüchen 2 und 3, dadurch gekennzeichnet, dass in der Abzweigung der Speiseleitung (60) vom Förderschacht (35) eine schwenkbare Klappe (46) angeordnet ist.

7. Anlage nach den Ansprüchen 2 und 4, dadurch gekennzeichnet, dass nach der Abzweigung der Speiseleitung (160) vom Förderschacht (135) im untersten Teil (133) der Speiseleitung eine Schleuse (140) angeordnet ist.

8. Anlage nach Anspruch 1, dadurch gekennzeichnet, dass der Austragungsschacht (45,145) ein Entwässerungsteilstück (45′,145′) aufweist.

9. Anlage nach Anspruch 1, dadurch gekennzeichnet, dass das Rührwerk (75) über ein Hebelgestänge (76′) mit einem Kolbenantrieb (76) schrittweise getrieben ist.

## Claims

1. Plant for the utilization of organic solids whilst obtaining compost and biogas, comprising a fermenter (70,170) for fermenting organic solids provided with a filling connection (64,164), a discharge connection (57,157), as well as a stirrer (75,175) rotatable about its longitudinal axis, as well as a crushing apparatus (20) associated with the fermenter, characterized in that the horizontal fermenter (70,170) can be filled with fresh material by means of a heated feed line (60,160) and by means of a feed shaft connected to the discharge connection (57,157) of the fermenter in a direct or indirect manner by means of a lock (30,40, 130,140) fermenting material can be added to the fresh material in the feed line, so that overall a closed process circuit is formed, the feed shaft (35,135) passing into a discharge shaft (45,145) of a discharge means.

2. Plant according to claim 1, characterized in that the bottom area (33,133) of the feed line (60,160) forms a branch of the feed shaft (35,135).

3. Plant according to claim 2, characterized in that a fresh material supply (27) directly introduces into the feed line (60) the fresh material prior to the branching to the feed line (60) via the feed shaft (35).

4. Plant according to claim 2, characterized in that a fresh material supply (127) introduces the fresh material directly into the feed line (60) after the branching of the bottom area (133) of the feed line from the feed shaft.

5. Plant according to claim 1, characterized in that the feed shaft (35,135) contains a hydraulically operable feed element (39) by means of which a fermenting material produced in the fermenter (70,170) can be transported as a bacterial inoculum in the feed shaft (35,135).

6. Plant according to claims 2 and 3, characterized in that a tiltable flap (46) is located in the branch of the feed line (60) from the feed shaft (35).

7. Plant according to claims 2 and 4, characterized in that a lock (140) is located in the bottom part (133) of the feed line following the branching of the feed line (160) from the feed shaft (135).

8. Plant according to claim 1, characterized in that the discharge shaft (45,145) has a draining section (45′,145′).

9. Plant according to claim 1, characterized in that the stirrer (75) is driven in stepwise manner with a piston drive (76) by means of a leverbar (76′).

## Revendications

1. Dispositif pour la valorisation de matières organiques avec obtention de compost et de biogaz, comprenant un fermenteur (70,170) muni d'une tubulure de remplissage (64,164), d'une tubulure d'évacuation (57,157), ainsi que d'un mélangeur (75,175) mobile autour de son axe longitudinal, pour la fermentation de matières organiques et un dispositif de broyage (20) relié au fermenteur, caractérisé en ce que, par une conduite d'alimentation chauffée (60,160), le fermenteur horizontal (70,170) peut être rempli de produit frais et, par une cheminée d'extraction raccordée à la tubulure d'évacuation (57,157) du fermenteur, directement ou indirectement au moyen de sas (30,40, 130,140), du produit de fermentation peut être ajouté au produit frais dans la conduite d'alimentation, de telle sorte qu'il se forme un cycle opératoire fermé au total sur lui-même, tandis que la cheminée d'évacuation (35,135) débouche dans une cheminée de déversement (45,145) d'un dispositif de déversement.

2. Dispositif selon la revendication 1, caractérisé en ce que la partie la plus basse (33,133) de la conduite d'alimentation (60,160) forme une dérivation de la cheminée d'évacuation (35,135).

3. Dispositif selon la revendication 2, caractérisé en ce qu'une arrivée de produit frais (27) envoie le produit frais avant la dérivation vers la conduite d'alimentation (60) via la cheminée d'évacuation (35) indirectement dans la conduite d'alimentation (60).

4. Dispositif selon la revendication 2, caractérisé en ce qu'une arrivée de produit frais (127) fait passer le produit frais, après la dérivation de la partie la plus basse (133) de la conduite d'alimentation, directement de la cheminée d'évacuation dans la conduite d'alimentation (60).

5. Dispositif selon la revendication 1, caractérisé en ce que dans la cheminée d'évacuation (35,135) est disposé un élément d'extraction à commande hydraulique (39), au moyen duquel un produit de fermentation obtenu dans le fermenteur (70,170) peut être transporté en tant que produit d'inoculation de bactéries dans la cheminée d'évacuation (35,135).

6. Dispositif selon l'une des revendications 2 et 3, caractérisé en ce que dans la dérivation de la conduite d'alimentation (60) de la cheminée d'évacuation (35) est disposé un clapet orientable (46).

7. Dispositif selon l'une des revendications 2 et 4, caractérisé en ce qu'après la dérivation de la conduite d'alimentation (160) de la cheminée d'évacuation (135) est disposé un sas (140) dans la partie la plus basse (133) de la conduite d'alimentation.

8. Dispositif selon la revendication 1, caractérisé en ce que la cheminée de déversement (45,145) comporte une portion pour la déshydratation (45′,145′).

9. Dispositif selon la revendication 1, caractérisé en ce que l'agitateur (75) est actionné pas à pas par l'intermédiaire d'une tige formant levier (76′) avec un entraînement par piston (76).
